(19) **Europäisches Patentamt**

**European Patent Office**

**Office européen des brevets**

(11) Publication number: **0 362 321 B1**

(12) # EUROPEAN PATENT SPECIFICATION

(45) Date of publication of patent specification: **14.07.93**

(51) Int. Cl.⁵: **A61K 9/06, A61K 47/00**

(21) Application number: **89902873.2**

(22) Date of filing: **08.02.89**

(86) International application number:
**PCT/US89/00451**

(87) International publication number:
**WO 89/06964 (10.08.89 89/18)**

(54) **OPHTHALMIC SUSPENSIONS.**

(30) Priority: **08.02.88 US 153762**
**25.01.89 US 301114**

(43) Date of publication of application:
**11.04.90 Bulletin 90/15**

(45) Publication of the grant of the patent:
**14.07.93 Bulletin 93/28**

(84) Designated Contracting States:
**AT BE CH DE FR GB IT LI LU NL SE**

(56) References cited:
EP-A- 2 007 091     US-A- 3 947 573
US-A- 4 271 143     US-A- 4 474 751
US-A- 4 478 818     US-A- 4 615 697

**No further relevant documents have been disclosed.**

(73) Proprietor: **INSITE VISION, INC.**
**965 Atlantic Avenue**
**Alameda, CA 94501(US)**

(72) Inventor: **CHANDRASEKARAN, Santosh,**
Kumar
14 Magee Court
Moraga, CA 94556(US)
Inventor: **ARCHIBALD, Roy, Duane**
34225 Whitehead Lane
Fremont, CA 94536(US)
Inventor: **DAVIS, Jeffrey, Paul**
2751 Chamberlain Avenue
Madison, WI 53705(US)
Inventor: **SU, Yansheng**
c/o Dr Jiang Shouli,Dept of Math,Shandong
Unvty,Jinan,Shandong,250100 P.R(CH)
Inventor: **ROBINSON, Joseph, R.**
41 Chequamegon Bay
Madison, WI 53719(US)

(74) Representative: **Sexton, Jane Helen et al**
**J.A. Kemp & Co. 14 South Square Gray's Inn**
**London WC1R 5LX (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.6/3.3.1)

EP 0 362 321 B1

**Description**

<u>FIELD OF THE INVENTION</u>

This invention relates to new topical ophthalmic medicament delivery systems and to methods of preparing them. More particularly, this invention relates to new topical ophthalmic medicament delivery systems comprising aqueous suspensions of particular lightly crosslinked polymers of acrylic acid or the like, which suspensions also contain an ophthalmic medicament. Such suspensions are easily administrable to the eye in drop form, and hence can be administered by or to a patient with a greater degree of comfort than either hitherto available petrolatum-based ophthalmic ointments or ophthalmic formulations containing the same or similar polymers in the form of aqueous, highly viscous gels or anhydrous suspensions or emulsions. The novel aqueous suspensions of this invention, once they have been dropped into the eye, come into contact with the eye's tear fluid, rapidly gel in situ to a substantially greater viscosity than that of the originally-introduced suspension and remain in place for prolonged periods of time. Sustained release of the medicament contained in the suspension - and now entrapped within the more viscous gel formed in the eye - then takes place.

<u>BACKGROUND OF THE INVENTION</u>

Medicaments have been administered to the eye in eyedrops, ointments or creams, in gelatin lamellae or other biologically soluble or insoluble films or sheets, by dispensing ocular inserts, as suspensions or emulsions in non-aqueous vehicles and in highly viscous aqueous gels. The disadvantages associated with each of these ophthalmic drug delivery systems are well known. Eyedrops in the form of aqueous solutions or suspensions are rapidly washed away by the eye's tear fluid. Ointments or creams blur the vision, and also have comparatively short residence times in the eye. Gelatin lamellae or other films or sheets, ocular inserts and non-aqueous suspensions and emulsions all can cause immediate pain and continuing discomfort and can also interfere with vision. Highly viscous aqueous gels, such as those disclosed in Schoenwald et al. U.S. Patents Nos. 4,271,143 and 4,407,792, issued June 2, 1981 and October 4, 1983, respectively, are difficult to administer so as to provide consistent, accurate dosages and may be uncomfortable to administer as well.

The Schoenwald et al. patents disclose that crosslinked carboxyl-containing polymers of the same general type as those employed in practicing this invention can be used in their ophthalmic drug delivery systems. Such systems are, however, formulated as either highly viscous aqueous gels or anhydrous suspensions and administered in those forms. Neither acrylic acid polymer-containing ophthalmic drug delivery systems formulated as aqueous suspensions capable of being administered in dropwise fashion nor any means by which such aqueous suspensions could be prepared are disclosed in the Schoenwald et al. patents.

A controlled release treatment composition that may be placed in the precorneal pocket of the eye containing a treating agent and a "bioadhesive" is disclosed in Robinson, U.S. Patent No. 4,615,697, issued October 7, 1986. The bioadhesive is described as a water-swellable, although water-insoluble, fibrous, cross-linked carboxy-functional polymer with a plurality of repeating units in which about at least 80 percent thereof contain at least one carboxy functionality and a cross-linking agent (0.05 to 1.5 percent) that is substantially free of polyalkenyl polyether. The bioadhesive is sized to, at the maximum, to pass through a sieve screen having a 10 mesh (U.S. Standard Sieve Series), that is, a 2000 $\mu$m opening, to minimize visual impairment. The viscosity, osmolality, and pH of the composition are not indicated.

An ophthalmic gel composition that is an aqueous solution of a carboxy vinyl polymer, a water-soluble basic substance, and an ophthalmic drug is taught in Toko Yakuhin Kogyo K.K., United Kingdom Patent Application GB 2,007,091A, published May 16, 1979. The gel has a pH of 5 to 8 and a viscosity of 1,000 to 100,000 centipoises (1 to 100 Pas) at 20°C. It is stated that adding a small amount of sodium chloride or an aqueous solution thereof to the gel causes it to convert to a liquid with a great reduction in viscosity. Contact with tear fluid will cause also a great reduction in viscosity.

It is therefore an object of this invention to provide new topical ophthalmic medicament delivery systems.

It is also an object of this invention to provide new topical ophthalmic medicament delivery systems that are easily administrable to the eye in drop form.

A further object of this invention is to provide new topical ophthalmic medicament delivery systems that are easily administrable in drop form and which comprise aqueous suspensions of particular lightly crosslinked polymers of acrylic acid or the like containing an ophthalmic medicament.

2

Yet another object of this invention is to provide new topical ophthalmic medicament delivery systems that are easily administrable in drop form and, after coming into contact with the eye's tear fluid, rapidly gel in the eye to a substantially greater viscosity than the viscosity of the administered drop.

A still further object of this invention is to provide methods of preparing these new topical ophthalmic medicament delivery systems.

The delivery system may be used to provide a method of administering new topical ophthalmic medicament delivery systems that are easily administrable in drop form, which method encompasses the treatment of "dry eye" by supplementing tear fluid.

These and other objects, as well as the nature, scope and utilization of this invention, will become readily apparent to those skilled in the art from the following description and the appended claims.

## SUMMARY OF THE INVENTION

Lightly crosslinked polymers containing predominantly carboxyl-containing monomers, such as Carbopol (trademark, The B.F. Goodrich Company) polymers, and preferably ones prepared by suspension or emulsion polymerizing acrylic acid or the like and a crosslinking agent such as divinyl glycol (3,4-dihydroxyl-1,5-hexadiene) or the like to an average dry particle size of not more than 50 $\mu$m in equivalent spherical diameter, are formulated with an ophthalmic medicament into suspensions in aqueous medium in which the amount of polymer, the pH, and the osmotic pressure (osmolality or tonicity) are within the ranges given hereinbelow. Such suspensions provide topical ophthalmic medicament delivery systems having suitably low viscosities that permit them to be easily administered to the eye in drop form, and hence be comfortably administrable in consistent, accurate dosages. These suspensions rapidly gel in the eye after coming into contact with the eye's tear fluid to a substantially greater viscosity than that of the originally-introduced suspension and thus remain in place over prolonged periods of time to provide comfortable and sustained release of the ophthalmic medicament.

## DETAILED DESCRIPTION OF THE INVENTION

The lightly crosslinked polymers of acrylic acid or the like used in practicing this invention are, in general, well known in the art. In a preferred embodiment such polymers are ones prepared from at least 50%, preferably 90%, and more preferably from 95% to 99.9% by weight, based on the total weight of monomers present, of one or more carboxyl-containing monoethylenically unsaturated monomers. Acrylic acid is the preferred carboxyl-containing monoethylenically unsaturated monomer, but other unsaturated, polymerizable carboxyl-containing monomers, such as methacrylic acid, ethacrylic acid, $\beta$-methylacrylic acid (crotonic acid), cis-$\alpha$-methylcrotonic acid (angelic acid), trans-$\alpha$-methylcrotonic acid (tiglic acid), $\alpha$-butylcrotonic acid, $\alpha$-phenylacrylic acid, $\alpha$-benzylacrylic acid, $\alpha$-cyclohexylacrylic acid, $\beta$-phenylacrylic acid (cinnamic acid), coumaric acid (o-hydroxycinnamic acid), umbellic acid (p-hydroxycoumaric acid), and the like can be used in addition to or instead of acrylic acid.

Such polymers are crosslinked by using a small percentage, i.e., from 0.1% to 5%, and preferably from 0.2% to 1%, based on the total weight of monomers present, of a polyfunctional crosslinking agent. Included among such crosslinking agents are non-polyalkenyl polyether difunctional crosslinking monomers such as divinyl glycol; 2,3-dihydroxyhexa-1,5-diene; 2,5-dimethyl-1,5-hexadiene; divinylbenzene; N,N-diallylacrylamide; N,N-diallylmethacrylamide and the like. Also included are polyalkenyl polyether crosslinking agents containing two or more alkenyl ether groupings per molecule, preferably alkenyl ether groupings containing terminal $H_2C = C<$ groups, prepared by etherifying a polyhydric alcohol containing at least four carbon atoms and at least three hydroxyl groups with an alkenyl halide such as allyl bromide or the like, e.g., polyallyl sucrose, polyallyl pentaerythritol, or the like; see, e.g., Brown U.S. Patent No. 2,798,053. Diolefinic non-hydrophilic macromeric crosslinking agents having molecular weights of from 400 to 8,000, such as insoluble di-and polyacrylates and methacrylates of diols and polyols, diisocyanate-hydroxyalkyl acrylate or methacrylate reaction products, and reaction products of isocyanate terminated prepolymers derived from polyester diols, polyether diols or polysiloxane diols with hydroxyalkylmethacrylates, and the like, can also be used as the crosslinking agents; see, e.g., Mueller et al. U.S. Patents Nos. 4,192,827 and 4,136,250.

The lightly crosslinked polymers can of course be made from a carboxyl-containing monomer or monomers as the sole monoethylenically unsaturated monomer present, together with a crosslinking agent or agents. They can also be polymers in which up to 40%, and preferably from 0% to 20% by weight, of the carboxyl-containing monoethylenically unsaturated monomer or monomers has been replaced by one or more non-carboxyl-containing monoethylenically unsaturated monomers containing only physiologically and

3

ophthalmologically innocuous substituents, including acrylic and methacrylic acid esters such as methyl methacrylate, ethyl acrylate, butyl acrylate, 2-ethyl-hexylacrylate, octyl methacrylate, 2-hydroxyethyl-methacrylate, 3-hydroxypropylacrylate, and the like, vinyl acetate, N-vinylpyrrolidone, and the like; see Mueller et al. U.S. Patent No. 4,548,990 for a more extensive listing of such additional monoethylenically unsaturated monomers. Particularly preferred polymers are lightly crosslinked acrylic acid polymers wherein the crosslinking monomer is 2,3-dihydroxyhexa-1,5-diene or 2,3-dimethylhexa-1,5-diene.

The lightly crosslinked polymers used in practicing this invention are preferably prepared by suspension or emulsion polymerizing the monomers, using conventional free radical polymerization catalysts, to a dry particle size of not more than 50 μm in equivalent spherical diameter; e.g., to provide dry polymer particles ranging in size from 1 to 30 μm, and preferably from 3 to 20 μm, in equivalent spherical diameter. In general, such polymers will range in molecular weight estimated to be 250,000 to 4,000,000, and preferably from 500,000 to 2,000,000.

Aqueous suspensions formulated in accordance with this invention containing polymer particles prepared by suspension or emulsion polymerization whose dry particle size is appreciably larger than 50 μm in equivalent spherical diameter are less comfortable when administered to the eye than suspensions otherwise identical in composition containing polymer particles whose equivalent spherical diameters are, on the average, below 50 μm. It has been discovered, furthermore, that lightly crosslinked polymers of acrylic acid or the like prepared to a dry particle size appreciably larger than 50 μm in equivalent spherical diameter and then reduced in size, e.g., by mechanically milling or grinding, to a dry particle size of not more than 50 μm in equivalent spherical diameter do not work as well as polymers made from aqueous suspensions as taught by this invention. While we do not wish to be bound by any theory or mechanism advanced to explain the functioning of this invention, one possible explanation for the difference of such mechanically milled or ground polymer particles as the sole particulate polymer present is that grinding disrupts the spatial geometry or configuration of the larger than 50 μm lightly crosslinked polymer particles, perhaps by removing uncrosslinked branches from polymer chains, by producing particles having sharp edges or protrusions, or by producing ordinarily too broad a range of particle sizes to afford satisfactory delivery system performance. A broad distribution of particle sizes will impair the viscosity-gelation relationship. In any event, such mechanically reduced particles are less easily hydratable in aqueous suspension than particles prepared to the appropriate size by suspension or emulsion polymerization, and also are less able to gel in the eye under the influence of tear fluid to a sufficient extent and are less comfortable once gelled than gels produced in the eye using the aqueous suspensions of this invention. However, up to 40% by weight, e.g., from 0% to over 20% by weight, based on the total weight of lightly crosslinked particles present, of such milled or ground polymer particles can be admixed with solution or emulsion polymerized polymer particles having dry particle diameters of not more than 50 μm when practicing this invention. Such mixtures will also provide satisfactory viscosity levels in the ophthalmic medicament delivery systems and in the in situ gels formed in the eye coupled with ease and comfort of administration and satisfactory sustained release of the medicament to the eye, particularly when such milled or ground polymer particles, in dry form, average from 0.01 to 30 μm, and preferably from 1 to 5 μm, in equivalent spherical diameter.

In the most preferred embodiment of the invention, the particles have a narrow particle size distribution. The use of a monodisperse particle will give maximum viscosity and an increased eye residence time of the ophthalmic medicament delivery systems for a given particle size. Monodisperse particles having a particle size of 30 μm and below are most preferred. Good particle packing is aided by a narrow particle size distribution.

The aqueous suspensions of this invention will contain amounts of lightly crosslinked polymer particles ranging from 0.1% to 6.5% by weight, and preferably from 0.5% to 4.5% by weight, based on the total weight of the aqueous suspension. They will preferably be prepared using pure, sterile water, preferably deionized or distilled, having no physiologically or ophthalmologically harmful constituents, and will be adjusted to a pH of from 3.0 to 6.5, and preferably from 4.0 to 6.0, using any physiologically and ophthalmologically acceptable pH adjusting acids, bases or buffers, e.g., acids such as acetic, boric, citric, lactic, phosphoric, hydrochloric, or the like, bases such as sodium hydroxide, sodium phosphate, sodium borate, sodium citrate, sodium acetate, sodium lactate, THAM (trishydroxymethylamino-methane), or the like and salts and buffers such as citrate/dextrose, sodium bicarbonate, ammonium chloride and mixtures of the aforementioned acids and bases.

When formulating the aqueous suspensions of this invention, their osmotic pressure ( $\pi$ ) will be adjusted to from 10 milliosmolar (mOsM) to 400 mOsM, and preferably from 100 to 250 mOsM, using appropriate amounts of physiologically and ophthalmologically acceptable salts. Sodium chloride is preferred to approximate physiologic fluid, and amounts of sodium chloride ranging from 0.01% to 1% by weight, and preferably from 0.05% to 0. 45% by weight, based on the total weight of the aqueous suspension, will give osmolalities within the above-stated ranges. Equivalent amounts of one or more salts made up of cations such as potassium, ammonium and the like and anions such as chloride, citrate, ascorbate, borate, phosphate, bicarbonate, sulfate, thiosulfate, bisulfite and the like, e.g., potassium chloride, sodium thiosulfate, sodium bisulfite, ammonium sulfate, and the like can also be used in addition to or instead of sodium chloride to achieve osmolalities within the above-stated ranges.

The amounts of lightly crosslinked polymer particles, the pH, and the osmotic pressure chosen from within the above-stated ranges will be correlated to give aqueous suspensions having viscosities ranging from 1,000 to 30,000 centipoise (1 to 30 Pas), and preferably from 5,000 to 20,000 centipoise (5 to 20 Pas), as measured at room temperature (about 25°C) using a Brookfield Digital LVT Viscometer equipped with a number 25 spindle and a 13R small sample adapter at 12 rpm. Such suspensions will gel on contact with tear fluid to give gels having viscosities estimated to range from 75,000 to 500,000 centipoise (75 to 500 Pas), e.g., from 200,000 to 300,000 centipoise (200 to 300 Pas), measured as above, depending on pH as observed, for example, from pH-viscosity curves. This effect is noted by observing a more viscous drop on the eye as a set cast. The cast, after setting, can be easily removed.

The viscous gels that result from fluid eyedrops delivered by means of the aqueous suspensions of this invention have residence times in the eye ranging from 2 to 12 hours, e.g., from 3 to 6 hours. The medicaments contained in these drug delivery systems will be released from the gels at rates that depend on such factors as the drug itself and its physical form, the extent of drug loading and the pH of the system, as well as on any drug delivery adjuvants, such as ion exchange resins compatible with the ocular surface, which may also be present. For fluorometholone, for example, release rates in the rabbit eye in excess of four hours, as measured by fluorometholone contained in the aqueous humor, have been observed.

Medicaments -- substances used in treating or ameliorating a disease or medical condition -- including drugs intended to treat therapeutically the eye itself or the tissues surrounding the eye and drugs administered via the ophthalmic route to treat therapeutically a local condition other than one involving the eye, will typically be incorporated in the topical delivery systems of this invention in therapeutically active amounts comparable to amounts administered in other dosage forms, usually in amounts ranging from 0.005% to 10% by weight, and preferably from 0.01% to 5% by weight, based on the total weight of the formulation. Thus, for example, from 0.01% to 1% by weight of the anti-inflammatory steroid fluorometholone can be administered in this manner.

An illustrative but by no means exhaustive listing of such medicaments includes antibiotics, antivirals, steroids, including anti-inflammatory agents, peptides, polypeptides, cardiotonics, antihypertensives, antiallergics, alpha- and beta-adrenergic blocking agents, ophthalmic medicaments such as anticataract agents, antiglaucoma agents and ophthalmic anti-inflammatory agents, ophthalmic lubricating agents, ophthalmic topical or regional anesthetic agents, etc. Specific medicaments that can be used in the present invention include drugs such as pilocarpine, idoxuridine, carbachol, bethanechol, timolol, atenolol, labetolol, metoprolol, nadolol, oxprenolol, pindolol, sotalol, betaxolol, acebutolol, alprenolol, levo-bunolol, p-aminoclonidine, dipivefrin, tetracycline, epinephrine, phenylephrine, eserine, phospholine, aceclidine, demecarium, cyclopentolate, homatropine, scopolamine, nitroglycerin, ethacrynic acid, furosemide, amiloride, chlortetracycline, bacitracin, neomycin, polymyxin, polymyxin B, gramicidin, oxytetracycline, chloramphenicol, gentamycin, penicillins, erythromycin, sulfacetamide, tobramycin, trospectomycin, vancomycin, ciprofloxacin, perfloxacin, olfloxacin, enoxacin, naphazoline hydrochloride, clindamycin, isofluorophate, fluorometholone, dexamethasone, hydrocortisone, fluorocinolone, medrysone, prednisolone, prednisolone acetate, methylprednisolone, fluticasone propionate, betamethasone, triamcinolone, estradiol, ibuprofen, flurbiprofen, naproxen, esters of ibuprofen, flurbiprofen, and naproxen; ketorolac, suprofen, interferons, cromolyn, gancyclovir, aminozolamide, alltrans-retinoic acid (Vitamin A) and the nontoxic, pharmaceutically acceptable salts thereof. Pro-drug counterparts are also within the scope of the present invention. Ophthalmic lubricating agents are materials capable of inducing natural lacrimation or creating artificial lacrimation and include, for example, polyvinylalcohol, cellulose polymers such as hydroxypropyl methyl cellulose, polylactams such as polyvinylpyrrolidone and the like. "Dry eye" formulations that comprise pure water and a lightly crosslinked polymer of the type described hereinabove in an amount within the range also set forth hereinabove, hypotonic in saline and thus having the requisite osmotic pressure but at a pH of 6.5 to 8, are also contemplated as being within the scope of this invention. Topical or regional anesthetic agents include ones used during ophthalmic surgery or other ophthalmic procedures, such as lidocaine, cocaine, benoxinate, dibucaine, proparacaine, tetracaine, etidocaine, procaine, hexylcaine, bupivacaine, mepivacaine, prilocaine, chloroprocaine, and the like.

The term "pharmaceutically acceptable salt" refers to those salts of the parent compound that do not significantly or adversely affect the pharmaceutical properties (e.g., toxicity, efficacy, etc.) of the parent compound. Pharmaceutically acceptable salts administerable by means of the aqueous suspensions of this invention include, for example, chloride, iodide, bromide, hydrochloride, acetate, nitrate, stearate, pamoate, phosphate and sulfate salts. It is sometimes desirable to use an appropriate salt form of the medicament that increases the water solubility or polar characteristics of the free drug.

The aqueous suspension topical ophthalmic medicament delivery systems of this invention can be formulated in any of several ways. For example the drug, the lightly crosslinked polymer particles, and the osmolality-adjusting salt can be pre-blended in dry form, added to all or part of the water, and stirred vigorously until apparent polymer dispersion is complete, as evidenced by the absence of visible polymer aggregates. Sufficient pH adjusting agent is then added incrementally to reach the desired pH, and more water to reach 100 percent formula weight can be added at this time, if necessary. Another convenient method involves adding the drug to about 95 percent of the final water volume and stirring for a sufficient time to saturate the solution. Solution saturation can be determined in known manner, e.g., using a spectrophotometer. The lightly crosslinked polymer particles and the osmolality-adjusting salt are first blended in dry form and then added to the drug-saturated suspension and stirred until apparent polymer hydration is complete. Following the incremental addition of sufficient pH adjusting agent to reach the desired pH, the remainder of the water is added, with stirring, to bring the suspension to 100 percent formula weight.

These aqueous suspensions can be packaged in preservative-free, single-dose non-reclosable containers. This permits a single dose of the medicament to be delivered to the eye one drop at a time, with the container then being discarded after use. Such containers eliminate the potential for preservative-related irritation and sensitization of the corneal epithelium, as has been observed to occur particularly from ophthalmic medicaments containing mercurial preservatives. Multiple-dose containers can also be used, if desired, particularly since the relatively low viscosities of the aqueous suspensions of this invention permit constant, accurate dosages to be administered dropwise to the eye as many times each day as necessary. In those suspensions where preservatives are to be included, suitable preservatives are chlorobutanol, Polyquat, benzalkonium chloride, cetyl bromide, and the like.

In order that those skilled in the art can more fully understand this invention, the following examples are set forth. These examples are given solely for purposes of illustration, and should not be considered as expressing limitations unless so set forth in the appended claims.

## EXAMPLE I

A pre-blend was prepared by dry-blending together 0.10 weight percent of fluorometholone (11$\beta$,17$\alpha$-dihydroxy-9$\alpha$-fluoro-6$\alpha$-methylpregna-1,4-diene-3,20-dione), 1.25 weight percent of Carbopol 976 (formerly known as Carbopol EX 55) (a carboxyl-containing polymer prepared by suspension polymerizing acrylic acid and divinyl glycol; The B.F. Goodrich Company) having a particle size of 5 $\mu$m, and 0.15 weight percent of sodium chloride. This pre-blend was added to 80 weight percent of deionized water in a vessel and stirred at 20 rpm at about 25°C for 12 hours. At this point apparent polymer dispersion was complete as evidenced by the absence of visible polymer aggregates.

The resulting aqueous drug-containing suspension was then titrated with 10N aqueous sodium hydroxide to pH 4.53; following which additional deionized water was added, with stirring, to bring the final formulation weight to 100 percent. The final aqueous suspension had an osmolality of approximately 50 mOsM and a viscosity of approximately 12,000 centipoise (12 Pas) as measured at 25°C on a Brookfield Digital LVT Viscometer equipped with a number 25 spindle and a 13R small sample adapter at 12 rpm.

## EXAMPLE II

Fluorometholone, 0.10 weight percent, was added to 80 weight percent of deionized water in a vessel and stirred at 50 rpm at 25°C for 24 hours to give a saturated aqueous suspension of the drug. Carbopol 976 polymer having a 5 $\mu$m particle size, 1.40 weight percent, and 0.25 weight percent of sodium chloride were blended in dry form and this blend was then added to the drug-saturated suspension, with stirring, at 20 rpm at 25°C for 12 hours.

The resulting aqueous drug-containing suspension was then titrated with 10N aqueous sodium hydroxide to pH 4.49, following which additional deionized water was stirred into the suspension to bring the final formulation weight to 100 percent. The final aqueous suspension had an osmolality of approximately 90 mOsM and a viscosity of approximately 18,000 centipoise(18 Pas), measured as in Example I.

**EXAMPLES III - VIII**

These examples relate to the preparation of "dry eye" formulations (Examples III - V) and pilocarpine hydrochloride formulations (Examples VI - VIII) of the present invention. For each example, NaCl and Carbopol 976, in the indicated weights, were dissolved in 100 g of distilled water using a mechanical mixer, after which the resulting formulation was sterilized at 121°C for 30 to 45 minutes. NaOH was then sterile-filtered to adjust the pH to the indicated range. In the pilocarpine examples, the pilocarpine hydrochloride was added by sterile filtration and the pH was adjusted following the sterilization. Carbopol 976 in all examples had a particle size of 5 μm.

| Dry Eye Formulations | | | |
|---|---|---|---|
| No. | Carbopol 976 (w/w %) | NaCl (w/w %) | pH |
| III | 1.05 | 0.175 | 5.6-5.8 |
| IV | 1.05 | 0.050 | 5.6-5.8 |
| V | 0.80 | 0.600 | 5.6-5.8 |

## Pilocarpine Hydrochloride Formulations

| No. | Pilocarpine (w/w %) | Carbopol 976 (w/w %) | NaCl (w/w %) | pH |
|---|---|---|---|---|
| VI | 1.0 | 2.0 | 0.1-0.9 | 5.2-5.8 |
| VII | 2.0 | 2.0 | 0.1-0.9 | 5.2-5.8 |
| VIII | 4.0 | 2.0 | 0.1-0.9 | 5.2-5.8 |

**EXAMPLE IX**

Various formulations were compounded to establish that the viscosity of the polymer solution is dependent on particle size. There were used Carbopol 976 and polycarbophyl, another polymer within the scope of the present invention. Polycarbophyl, as referred to here, is a polyacrylic acid polymer lightly cross-linked with divinylglycol, meeting the compendium specifications of the United States Pharmacopeia, and was obtained as an experimental sample from The B.F. Goodrich Company.

A polycarbophyl lot was sieved to ranges of greater than 105 μm, less than 105 μm, less than 105 but greater than 75 μm, and less than 75 but greater than 45 μm. A sample was also ground to a size of less than 10 μm.

The general formulation used for all was 1.05 w/w% polymer and 0.2 w/w% NaCl with a pH of 5.2-5.6. The correlation between particle size and viscosity is shown in the following table.

7

EP 0 362 321 B1

| Nominal Polymer | Viscosity (cps)+ [Pas] | (Dry) Particle Size (μm) |
|---|---|---|
| Carbopol 976 | 28,000 [ 28] | 5 |
| Polycarbophyl | 1,080 [1.08] | < 105 |
| Polycarbophyl | 19,800 [19.8] | < 10 |
| Polycarbophyl | 1,800 [1.8] | > 105 |
| Polycarbophyl | 2,800 [2.8] | > 75 and < 105 |
| Polycarbophyl | 9,200 [9.2] | > 45 and < 75 |
| 80 parts Carbopol 976/20 parts Polycarbophyl | 19,200 [19.2] | 5 / < 105 |
| 90 parts Carbopol 976/10 parts Polycarbophyl | 22,000 [22] | 5 / < 105 |

+ Measured at about 25°C using a Brookfield Digital LVT Viscometer equipped with a number 25 spindle and a 13R small sample adapter at 12 rpm.

**EXAMPLE X**

This example is directed to a fluoromethalone suspension within the scope of the present invention.

Fluoromethalone, 0.10 weight %, was added to 97 weight % of purified water in a vessel and stirred at high speed for 15 minutes to give a finely dispersed aqueous suspension of the drug. Carbopol 976 polymer having a dry particle size of 5 μm, 1.05 weight %, was added to the drug suspension with stirring and mixing was continued for a minimum of 15 minutes. After the 15-minute minimum time had elasped, 0.20 weight % of sodium chloride was added.

The resulting aqueous drug-containing suspension was sterilized at 121°C for 45 minutes. The suspension was cooled to about 50°C and a 10 N sodium hydroxide solution was then sterile filtered into the suspension with stirring to adjust the pH to 5.6-5.8. Additional purified water was sterile filtered into the suspension with stirring to bring the final formulation weight to 100%. The final aqueous suspension had an osmolality of approximately 150 mOsM, a viscosity of approximately 15,700 centipoise (15.7 Pas), measured at room temperature (about 25°C) using a Brookfield Digital LVT Viscometer equipped with a number 25 spindle and a 13R small sample adapter at 12 RPM, and a pH of about 5.6-5.8.

**EXAMPLE XI**

This example relates to a "dry eye"/tear substitute formulation.

Carbopol 976 polymer having a dry particle size of 5 μm, 0.8 weight %, was added to 97 weight % of purified water in a vessel and stirred at high speed for a minimum of 15 minutes. Sodium chloride, 0.6 weight %, was then added to the aqueous polymer suspension with stirring.

The resulting suspension was sterilized at 121°C for 45 minutes. The suspension was cooled to about 50°C and 10 N sodium hydroxide solution was then sterile filtered into the suspension with stirring to adjust the pH to 7.6-7.8. Additional purified water was sterile filtered into the suspension with stirring to bring the final formulation weight % to 100 percent. The final aqueous suspension had an osmolality of approximately 270 mOsM, a viscosity of approximately 3600 cps (3.6 Pas), measured as above, and a pH of about 7.6-7.8.

**Claims**

1. A sustained release topical ophthalmic medicament delivery system comprising:

an aqueous suspension at a pH of from 3 to 6.5 and an osmotic pressure of from 10 to 400 mOsM containing from 0.1% to 6.5% by weight, based on the total weight of the suspension, of a carboxyl-containing polymer prepared by polymerizing at least 50% by weight of one or more carboxyl-containing monoethylenically unsaturated monomers and from 0.1% to 5% by weight of a crosslinking agent, said weight percentages of monomers being based on the total weight of monomers polymerized,

said suspension having a viscosity of from 1,000 to 30,000 centipoise (1 to 30 Pas) and being administrable to the eye in drop form,

said polymer having an average particle size of not more than 50μm in equivalent spherical diameter and being lightly cross-linked such that although the suspension is deliverable in drop form, upon contact of the lower pH suspension with the higher pH tear fluid in the eye, the suspension is rapidly gellable to a substantially greater viscosity than the viscosity of the Suspension as originally

8

EP 0 362 321 B1

administered in drop form,
whereby the resulting more viscous gel can remain in the eye for sustained release of a medicament contained therein.

2. A topical ophthalmic medicament delivery system as in claim 1 containing an ophthalmic medicament.

3. A topical ophthalmic medicament delivery system as in claim 1 or 2 in which said polymer has a particle size of not more than 30 $\mu$m.

4. A topical ophthalmic medicament delivery system as claimed in claim 1, 2 or 3 in which said polymer is a monodispersion of particles.

5. A topical ophthalmic medicament delivery system as claimed in claim 1, 2 or 3 in which said polymer is one prepared from at least 90% by weight of one or more carboxyl-containing monoethylenically unsaturated monomers.

6. A topical ophthalmic medicament delivery system as claimed in claim 1, 2 or 3 in which said polymer is one prepared by suspension or emulsion polymerizing acrylic acid and a non-polyalkenyl polyether difunctional crosslinking agent to a particle size of not more than 50 $\mu$m in equivalent spherical diameter.

7. A topical ophthalmic medicament delivery system as claimed in claim 6 in which said crosslinking agent is divinyl glycol.

8. A topical ophthalmic medicament delivery system as in claim 7 in which said osmotic pressure is achieved using a physiologically and ophthalmologically acceptable salt in an amount of from 0.01% to 1% by weight, based on the total weight of the suspension.

9. A topical ophthalmic medicament delivery system as claimed in claim 8 in which said salt is sodium chloride.

10. A topical ophthalmic medicament delivery system as claimed in any one of claims 1 to 9 in which a medicament is present in an amount of from 0.005% to 10% by weight, based on the total weight of the suspension.

11. A topical ophthalmic medicament delivery system as claimed in claim 10 in which said medicament is fluorometholone.

12. A topical ophthalmic medicament delivery system as in claim 10 in which said medicament is pilocarpine.

13. Use of a topical ophthalmic medicament delivery system as defined in any one of the preceding claims in the manufacture of a medicament for delivery in the service of the eye.

14. A dry eye/tear substitute system administrable to the eye in drop form comprising an ophthalmic lubricating agent in an aqueous suspension containing from 0.1% to 6.5% by weight, based on the total weight of said suspension, of a lightly crosslinked carboxyl-containing polymer having a particle size of not more than 50 $\mu$m in equivalent spherical diameter, prepared by polymerizing at least 50% by weight of one or more carboxy-containing monoethylenically unsaturated monomers and from 0.1% to 5% by weight of a crosslinking agent, said weight percentages of monomers being based on the total weight of monomers polymerized, said suspension being at a pH of from 6.5 to 8.0 and an osmotic pressure of from 10 to 400 mOsM and having a viscosity of from 1,000 to 30,000 centipoise (1 to 30 Pas).

**Patentansprüche**

1. Trägersystem für ein topisches Augen-Depotmedikament, welches umfaßt:
Eine wässrige Suspension mit einem pH-Wert von 3 bis 6,5 und einem osmotischen Druck von 10 bis

9

400 mOsM, die 0,1 bis 6,5 Gew.-%, bezogen auf das Gesamtgewicht der Suspension, eines Carboxyl enthaltenden Polymers enthält, das durch Polymerisieren von mindestens 50 Gew.-% eines oder mehrerer Carboxyl enthaltender, monoethylenisch ungesättigter Monomeren und 0,1 bis 5 Gew.-% eines Vernetzungsmittels hergestellt wird, wobei sich die Gewichtsprozente der Monomeren auf das Gesamtgewicht der polymerisierten Monomeren beziehen,

wobei die Suspension eine Viskosität von 1.000 bis 30.000 Zentipoise (1 bis 30 Pa.s) aufweist und dem Auge in Tropfenform verabreichbar ist,

wobei das Polymer eine mittlere Teilchengröße von nicht mehr als 50 $\mu$m bei äquivalentem Kugeldurchmesser besitzt und leicht vernetzt ist, so daß die Suspension, obwohl in Tropfenform verabreichbar, dann, wenn sich die Suspension mit ihrem niedrigeren pH-Wert mit der Tränenflüssigkeit mit ihrem höheren pH-Wert im Auge verbindet, schnell zu wesentlich höherer Viskosität als der Viskosität der ursprünglich in Tropfenform verabreichten Suspension gelierfähig ist,

wobei das resultierende viskosere Gel zum Zwecke der Depotwirkung eines darin enthaltenen Medikaments im Auge verbleiben kann.

2. Trägersystem für ein topisches Augen-Depotmedikament nach Anspruch 1, welches ein ophthalmisches Medikament enthält.

3. Trägersystem für ein topisches Augen-Depotmedikament nach Anspruch 1 oder 2, worin das Polymer eine Teilchengröße von nicht mehr als 30 $\mu$m besitzt.

4. Trägersystem für ein topisches Augen-Depotmedikament nach Anspruch 1, 2 oder 3, worin das Polymer eine Monodispersion von Teilchen ist.

5. Tragersystem für ein topisches Augen-Depotmedikament nach Anspruch 1, 2 oder 3, worin das Polymer ein solches ist, das zu mindestens 90 Gew. -% aus einem oder mehreren Carboxyl enthaltenden, monoethylenisch ungesättigten Monomeren hergestellt ist.

6. Trägersystem für ein topisches Augen-Depotmedikament nach Anspruch 1, 2 oder 3, worin das Polymer ein solches ist, das durch Suspensions- oder Emulsionspolymerisation von Acrylsäure und einem nicht in Polyalcenylpolyether bestehendem, difunktionellen Vernetzungsmittel, zu einer Teilchengröße von nicht mehr als 50 $\mu$m bei äquivalentem Kugeldurchmesser, hergestellt ist.

7. Trägersystem für ein topisches Augen-Depotmedikament nach Anspruch 6, worin das Vernetzungsmittel Divinylglykol ist.

8. Trägersystem für ein topisches Augen-Depotmedikament nach Anspruch 7, worin der osmotische Druck unter Verwendung eines physiologisch und ophthalmologisch geeigneten Salzes in einer Menge von 0,01 bis 1 Gew.-%, bezogen auf das Gesamtgewicht der Suspension, erreicht wird.

9. Trägersystem für ein topisches Augen-Depotmedikament nach Anspruch 8, worin das Salz Natriumchlorid ist.

10. Trägersystem für ein topisches Augen-Depotmedikament nach einem der Ansprüche 1 bis 9, worin ein Medikament in einer Menge von 0,005 bis 10 Gew.-%, bezogen auf das Gesamtgewicht der Suspension, enthalten ist.

11. Tragersystem für ein topisches Augen-Depotmedikament nach Anspruch 10, worin das Medikament Fluorometholon ist.

12. Trägersystem für ein topisches Augen-Depotmedikament nach Anspruch 10, worin das Medikament Pilocarpin ist.

13. Verwendung eines Trägersystems für ein topisches Augen-Depotmedikament, wie in jedem der vorangegangenen Ansprüche definiert, bei der Herstellung eines Medikaments zur Verabreichung in das Auge.

**14.** Trockenes Auge/Tränenersatz-System, welches dem Auge in Tropfenform verabreichbar ist, umfassend ein ophthalmisches Gleitmittel in einer wässrigen Suspension, welche 0,1 bis 6,5 Gew.-%, bezogen auf das Gesamtgewicht der Suspension, eines leicht vernetzten, Carboxyl enthaltenden Polymers mit einer Teilchengröße von nicht mehr als 50 μm bei äquivalentem Kugeldurchmesser enthält, welches durch Polymerisieren von mindestens 50 Gew.-% eines oder mehrerer monoethylenisch ungesättigter Monomeren und von 0,1 bis 5 Gew.-% eines Vernetzungsmittels hergestellt wird, wobei sich die Gewichtsprozente der Monomeren auf das Gesamtgewicht der polymerisierten Monomeren beziehen, in welchem die Suspension einen pH-Wert von 6,5 bis 8,0, einen osmotischen Druck von 10 bis 400 mOsM und eine Viskosität von 1.000 bis 30.000 Zentipoise (1 bis 30 Pa.s) aufweist.

## Revendications

**1.** Système de libération, ayant une action prolongée, de médicament ophtalmique à usage local, comprenant :

une suspension aqueuse, ayant un pH de 3 à 6,5 et une pression osmotique de 10 à 400 mOsM, contenant de 0,1 % à 6,5 % en poids, par rapport au poids total de la suspension, d'un polymère carboxylé, préparé par polymérisation d'au moins 50 % en poids d'un ou de plusieurs monomères insaturés monoéthyléniques carboxylés, et de 0,1 à 5 % en poids d'un agent réticulant, lesdits pourcentages en poids des monomères étant calculés par rapport au poids total des monomères polymérisés,

ladite suspension ayant une viscosité de 1000 à 30 000 centipoises (1 à 30 Pa•s) et étant administrable dans l'oeil sous forme de gouttes,

ledit polymère ayant une taille moyenne de particules qui n'est pas supérieure à 50 μm, exprimée en diamètre sphérique équivalent, et étant faiblement réticulé, de sorte que, bien que la suspension soit administrée sous la forme de gouttes, par contact de la suspension de faible pH avec le fluide lacrymal de pH supérieur, la suspension se gélifie rapidement en une substance ayant une viscosité supérieure à celle de la suspension initialement administrée sous forme de gouttes,

ce qui permet au gel résultant, de viscosité élevée, de rester dans l'oeil pour permettre une libération prolongée du médicament qu'il contient.

**2.** Système de libération de médicament ophtalmique à usage local, selon la revendication 1, contenant un médicament ophtalmique.

**3.** Système de libération de médicament ophtalmique à usage local, selon la revendication 1 ou 2, dans lequel ledit polymère présente une taille de particules qui n'est pas supérieure à 30 μm.

**4.** Système de libération de médicament ophtalmique à usage local, selon la revendication 1, 2 ou 3, dans lequel ledit polymère est une monodispersion de particules.

**5.** Système de libération de médicament ophtalmique à usage local, selon la revendication 1, 2 ou 3, dans lequel ledit polymère est préparé à partir d'au moins 90 % en poids d'un ou de plusieurs monomères insaturés monoéthyléniques carboxylés.

**6.** Système de libération de médicament ophtalmique à usage local, selon la revendication 1, 2 ou 3, dans lequel ledit polymère est préparé par polymérisation en suspension ou en émulsion de l'acide acrylique et d'un agent de réticulation bifonctionnel polyéther non polyalcénylique, jusqu'à obtention d'une taille des particules non supérieure à 50 μm, exprimée en diamètre sphérique équivalent.

**7.** Système de libération de médicament ophtalmique à usage local, selon la revendication 6, dans lequel ledit agent de réticulation est le divinylglycol.

**8.** Système de libération de médicament ophtalmique à usage local, selon la revendication 7, dans lequel ladite pression osmotique est obtenue en utilisant un sel physiologiquement et ophtalmologiquement acceptable, dans une proportion de 0,01 % à 1 % en poids, par rapport au poids total de la suspension.

**9.** Système de libération de médicament ophtalmique à usage local, selon la revendication 8, dans lequel ledit sel est le chlorure de sodium.

**10.** Système de libération de médicament ophtalmique à usage local, selon les revendications 1 à 9, dans lequel le médicament est présent dans une proportion de 0,005 % à 10 % en poids, par rapport au poids total de la suspension.

**11.** Système de libération de médicament ophtalmique à usage local, selon la revendication 10, dans lequel ledit médicament est la fluorométholone.

**12.** Système de libération de médicament ophtalmique à usage local, selon la revendication 10, dans lequel ledit médicament est la pilocarpine.

**13.** Utilisation d'un système de libération de médicament ophtalmique à usage local, selon l'une quelconque des revendications précédentes, dans la fabrication d'un médicament destiné à l'administration en ophtalmologie.

**14.** Système de substitution pour yeux secs/larmes, administrable dans l'oeil sous forme de gouttes, comprenant un agent lubrifiant ophtalmique en suspension aqueuse, contenant de 0,1 % à 6,5 % en poids, par rapport au poids total de ladite suspension, d'un polymère carboxylé faiblement réticulé, ayant une taille de particules qui n'est pas supérieure à 50 $\mu$m, exprimé en diamètre sphérique équivalent, préparé par polymérisation d'au moins 50 % en poids d'un ou de plusieurs monomères insaturés monoéthyléniques, et 0,1 à 5 % en poids d'un agent de réticulation, lesdits pourcentages en poids des monomères étant calculés par rapport au poids total des monomères polymérisés, ladite suspension ayant un pH de 6,5 à 8,0 et une pression osmotique de 10 à 400 mOsM, et une viscosité de 1000 à 30 000 centipoises (1 à 30 Pa•s).